# EUROPEAN PATENT APPLICATION

(11) **EP 0 982 046 A1**
(43) Date of publication of application: **01.03.2000**
(21) Application number: 99306768.5
(22) Date of filing: 25.08.1999
(51) Int. Cl.: A61M 25/01

(54) **Clinical guide wire and method for producing the same**

(30) Priority: 28.08.1998 JP 24322898; 19.04.1999 JP 11094899
(71) Applicant: Piolax Inc., Yokohama-shi, Kanagawa-ken (JP)
(72) Inventor: Asano, Hiroyuki, Yokohama-shi, Kanagawa-ken (JP)
(74) Representative: Abbie, Andrew Kenneth

(57) **Abstract**

A clinical guide wire includes: a core made from an elastic material such that the tip section has a diameter smaller than that of the main section; a resin tube which covers at least part of the tip section of the core; and a hydrophilic resin film which covers the outer periphery of the resin tube. In the construction described above, the outer diameter of the portion covered with the resin tube and the hydrophilic resin film is smaller than the product outer diameter in the main section of the core.

## Description

### BACKGROUND OF THE INVENTION

### Field of the Invention

The present invention relates to a clinical guide wire which is used for guiding a catheter tip to a target position when inserting the catheter into tubular organs of humans such as blood vessels, ureters, bile ducts, tracheae, and the like.

### Related Art

In recent years, for examining and treating tubular organs of humans, such as blood vessels, ureters, bile ducts, tracheae, and the like, it is often practiced to administer a drug such as a contrast medium by insertion of a catheter or to collect a portion of tissue by a clamp or the like through a catheter. When a catheter is inserted, a relatively fine and flexible guide wire is first inserted into a tubular organ so that the tip of the guide wire reaches the target position, the catheter is then inserted along the outer periphery of the guide wire, and thereafter the guide wire is withdrawn.

Properties which are desired for the guide wire include a fineness ensuring smooth insertion of the guide wire into a target organ, tip flexibility in order not to injure the inner wall of the tubular organ, rigidity of the main section allowing the guide wire to be pushed into the tubular organ, slipperiness inside the catheter and the tubular organ, and prevention of the adhesion of thrombi.

As an example of such guide wires, Japanese Patent Application Laid-Open No. 10-146,390 discloses a guide wire comprising an ultra-elastic core, which comprises a main section, a tip section having a diameter smaller than that of the main section, and a transitional section between the main section and the tip section, a metal coil which is fixed to the tip section of the core and which is capable of imaging with X-rays, a synthetic resin coating member which covers at least part of the metal coil capable of imaging with X-rays and the core to thereby form a substantially smooth outer surface, and a hydrophilic lubricating layer which covers at least part of the synthetic resin coating member.

The guide wire in the related art, however, has been associated with a minor point. That is, if the synthetic resin coating member and the hydrophilic lubricating layer are provided on the entire surface of the core, these resin layers add to the thickness of the guide wire. Therefore, in order to maintain the fineness ensuring smooth insertion into a target tubular organ, the overall core necessarily needs to be so fine that the rigidity for the insertion of the core into the tubular organ becomes insufficient, thereby degrading the push ability.

Meanwhile, an attempt to form a synthetic resin coating member and a hydrophilic lubricating layer solely on the tip section of the core was encountered with a problem that the formation of the synthetic resin coating member on the core was very difficult. This is because none of such methods as extrusion forming, injection forming, and dipping made it possible to easily form a thin and smooth synthetic resin coating solely on the tip section of the core having a complex shape due to the attachment of a metal coil capable of imaging with X-rays and others.

### SUMMARY OF THE INVENTION

The present invention has been achieved with such points in mind.

It therefore is an object of the present invention to provide a clinical guide wire which has good push ability ensured by a sufficiently large outer diameter of the main section of the core as well as such properties as slipperiness and prevention of the adhesion of thrombi.

Another object is to provide a method for producing the above-mentioned clinical guide wire of a uniform quality in a stable manner.

In order to achieve the above-described object, a first aspect of the present invention is a clinical guide wire comprising: a core made from an elastic material such that the tip section has a diameter smaller than that of the main section; a resin tube which covers at least part of the tip section of the core; and a hydrophilic resin film which covers the outer periphery of the resin tube, wherein the outer diameter of the portion covered with the resin tube and the hydrophilic resin film is smaller than the product outer diameter in the main section of the core.

According to the first aspect of the present invention, covering the tip section of the core with a resin tube makes it possible to easily form a thin and smooth resin layer having a uniform thickness on the tip section of the core. The hydrophilic resin film formed on the surface of the resin tube ensures excellent slipperiness and prevention of the adhesion of thrombi. Furthermore, since the outer diameter of the portion covered with the resin tube and the hydrophilic resin film is equal to or smaller than the product outer diameter in the main section of the core, insertion into fine blood vessels becomes possible. Furthermore, since the rigidity can be maintained by providing a sufficiently large outer diameter to the main section of the core, push ability at the time of insertion is excellent.

A second aspect of the present invention, as it depends from the first aspect, is a clinical guide wire, wherein a metal coil impermeable to X-rays is attached to the outer periphery of the endmost tip of the core; and wherein the outer periphery of the metal coil is covered completely with the resin tube.

According to the above-described construction, the position of the tip section can be confirmed by the metal coil impermeable to X-rays without impairing the flexibility of the tip section, and the slipperiness of the tip section can be upgraded by completely covering the outer periphery of the metal coil with the resin tube.

A third aspect of the present invention, as it depends from the first aspect or the second aspect, is a clinical guide wire, wherein a metal coil impermeable to X-rays is attached to the outer periphery of the endmost tip of the core; and wherein at least the tip of the metal coil protrudes from the resin tube.

According to the above-described construction, the position of the tip section can be confirmed by the metal coil impermeable to X-rays, and the flexibility of the endmost tip section can be upgraded by protruding at least the tip of the metal coil from the resin tube.

A fourth aspect of the present invention, as it depends from one aspect among the first aspect to the third aspect, is a clinical guide wire, wherein the resin tube covers the tip section of the core such that the resin tube further protrudes from the endmost tip of the core.

According to the above-described construction, the slipperiness is provided by the resin tube, and the flexibility of the endmost tip section can be further upgraded by protruding the resin tube from the endmost tip of the core.

A fifth aspect of the present invention, as it depends from one aspect among the first aspect to the fourth aspect, is a clinical guide wire, wherein the main section of the core is covered with a hydrophobic resin film.

According to the above-described construction, it is possible to upgrade the slipperiness and prevention of adhesion of thrombi of the outer periphery of the main section of the core without the reduction of the outer diameter in the main section of the core.

A sixth aspect of the present invention, as it depends from one aspect among the first aspect to the fifth aspect, is a clinical guide wire, wherein a material impermeable to X-rays is blended into the resin tube for the core.

According to the above-described construction, the position of the tip section can be confirmed more easily by increasing the impermeability to X-rays in the tip section.

A seventh aspect of the present invention is a method for producing a clinical guide wire, the method comprising the steps of: (a) making a core from an elastic material such that the diameter of the tip section is smaller than that of the main section; (b) covering at least part of the tip section of the core with a resin tube; and (c) covering the outer periphery of the resin tube with a hydrophilic resin film.

According to the seventh aspect of the present invention, it is possible to easily form a smooth resin layer having a uniform thickness.

An eighth aspect of the present invention, as it depends from the seventh aspect, is a method for producing a clinical guide wire, wherein the resin tube which covers at least part of the tip section of the core is a resin tube swollen with a solvent; and wherein the method for producing a clinical guide wire further comprises the following step after the step (b) and before the step (c): evaporating the solvent to shrink-wrap the tip section of the core with the resin tube.

According to the eight aspect of the present invention, it is possible to tightly cover the tip section of the core made from an elastic material with a resin tube and therefore it is possible to easily form a smooth and thin resin layer having a uniform thickness on the core.

A ninth aspect of the present invention, as it depends from the seventh aspect, is a method for producing a clinical guide wire, the method further comprising the following step after the step (b) and before the step (c): bonding or fusing the resin tube to the tip section of the core.

According to the ninth aspect of the present invention, since a portion of the resin tube is fixed by bonding or fusion, the resin tube is stretched when the resin tube is covered with a hydrophilic resin film and, as a result, a smooth and thin covering having a uniform thickness is formed by the resin tube on the core.

A tenth aspect of the present invention, as it depends from one aspect among the seventh aspect to the ninth aspect, is a method for producing a clinical guide wire, the method further comprising the following step after the step (a) and before the step (b): attaching a metal coil impermeable to X-rays to the outer periphery of the endmost tip of the core, wherein the outer periphery of the metal coil is then covered with the resin tube in the following step (b).

According to the tenth aspect of the present invention, it is possible to easily form a smooth and thin synthetic resin layer having a uniform thickness on the outer periphery of the tip section of the core having a complex shape due to the attachment of the metal coil impermeable to X-rays.

### BRIEF DESCRIPTION OF THE ACCOMPANYING DRAWINGS

The above and further objects and novel features of the present invention will more fully appear from the following detailed description when the same is read in conjunction with the accompanying drawings, in which:
Fig. 1 is a cross-sectional view illustrating one embodiment of the clinical guide wire according to the present invention;
Fig. 2A, Fig. 2B, Fig. 2C, and Fig. 2D are process drawings illustrating an embodiment of the method for producing the clinical guide wire according to the present invention;
Fig. 3 is a cross-sectional view illustrating another embodiment of the clinical guide wire according to the present invention;
Fig. 4 is a cross-sectional view illustrating another embodiment of the clinical guide wire according to the present invention;
Fig. 5 is a cross-sectional view illustrating another embodiment of the clinical guide wire according to the present invention;
Fig. 6 is a cross-sectional view illustrating another embodiment of the clinical guide wire according to the present invention; and
Fig. 7 is a cross-sectional view illustrating yet another embodiment of the clinical guide wire according to the present invention.

### DETAILED DESCRIPTION OF THE PREFERRED EMBODIMENTS

There will be detailed below the preferred embodiments of the present invention with reference to the accompanying drawings. Like members are designated by like reference characters.

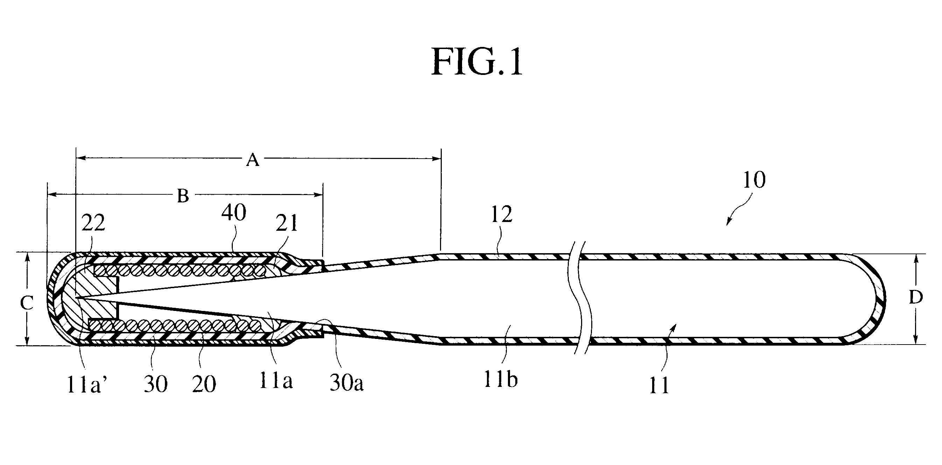

Fig. 1 illustrates one embodiment of the clinical guide wire according to the present invention. A guide wire indicated by a reference numeral 10 comprises a core 11, a metal coil 20 which is impermeable to X-rays and provided on the outer periphery of the tip section lla of the core 11, a resin tube 30 covering the tip section lla of the core 11 and the metal coil 20, and a hydrophilic resin film 40 covering the outer periphery of the resin tube 30.

The core 11 is made from an ultra-elastic material or an elastic material such as stainless steel, piano wires, and the like. Preferred examples of the ultra-elastic material include a Ni-Ti alloy, a Cu-Zn-X (X = Al, Fe, or the like) alloy, a Ni-Ti-X (X = Fe, Cu, V, Co, or the like) alloy, and others.

The above-mentioned alloys such as Ni-Ti alloys and others are widely known as shape-retentive alloys having a shape-retentive effect and an ultra-elastic (pseudo-elastic) effect. Among these alloys, an alloy having an ultra-elastic (pseudo-elastic) effect is suitable as a core material for the guide wire. This is because the alloy is characterized in that, even if a deforming strain over yield point is given, this alloy is restored to the original shape when the load is removed and thus exhibits a strong propensity to return to the original state after torsion and bending, unlike an ordinary metal material which exhibits a permanent deformation when a deforming strain over yield point is given. Preferably, the temperatures, at which the ultra-elastic (pseudo-elastic) effect is to be exhibited, are made equal to or lower than the temperatures inside the body of humans or animals.

As to the ultra-elastic (pseudo-elastic) property, reference can be made to JIS H 7001 issued from Japanese Standards Association.

The core 11 has a main section 11b and a tip section 11a having a diameter smaller than that of the main section 11b. It is preferable that the tip section 11a has a shape in which the diameter dwindles towards the endmost tip 11a'. For example, a tapered shape, a stepwise shape, or the like is preferred.

The length A of the tip section 11a whose diameter is smaller than that of the main section 11b is preferably about 150 to 700 mm. The whole length of the core 11, i.e., the length of the guide wire 10, is preferably about 1000 to 3000 mm. The diameter of the main section 11b of the core 11 is appropriately selected from a range of from 0.2 to 1.0 mm according to purposes.

In order to impart the slipperiness and the ability to prevent the adhesion of thrombi to the core 11, the outer periphery of the main section 11 is covered with a hydrophobic resin film 12. Preferred examples of the hydrophobic resin film 12 include a silicone resin, a fluorocarbon resin, and the like.

A metal coil 20, which is impermeable to X-rays, is attached on the outer periphery of the tip section lla of the core 11. According to this embodiment, the metal coil 20 is fixed to the tip section of the core 11 via soldering materials 21 and 22, wherein the soldering material 22 forms a round head which encloses the endmost tip 11a' of the core 11. Preferred examples of the material for the metal coil 20 impermeable to X-rays include gold, platinum, silver, bismuth, tungsten, and alloys containing these metals. Although the metal coil 20 impermeable to X-rays may be attached to the whole of the tip section 11a whose diameter is smaller than that of the main section 11b, the metal coil 20 impermeable to X-rays may be attached to a region from part of the tip section 11a to the endmost tip 11a'. The means of fixing the metal coil 20 to the core 11 is not limited to brazing with a soldering material. Other means, such as bonding with an adhesive, can also be adopted.

A resin tube 30 covers the tip section 11a of the core 11. Preferably, the region covered with the resin tube 30 is a portion having a prescribed length from the endmost tip 11a' of the corell and the outer periphery of the metal coil 20. The material for the resin tube 30 is not particularly limited so long as it can be covered with a hydrophilic resin film 40. Examples of the material for the resin tube 30 may include polyurethane, polyetherblockamide, polyethylene, polyvinyl chloride, polyester, polypropylene, polyamide, polystyrene, fluorocarbon resins, silicone resins, and the like. The resin tube 30 may contain a material impermeable to X-rays. Preferred examples of the material impermeable to X-rays include powders of such substances as bismuth, barium sulfate, and tungsten.

The length B of the resin tube 30 is preferably a length enough for covering the portion ranging from the endmost tip 11a' of the corell to the position short of the main section 11b. More specifically, the length of B is preferably 150 to 700 mm and more preferably 150 to 500 mm. Although the thickness of the resin tube 30 is not particularly limited, it is preferably about 0.5 to 50µm.

In this embodiment, preferable is a shape in which the tip of the resin tube 30 is closed so that it completely covers the endmost tip 11a' of the core 11 and the soldering material 22. However, the tip of the resin tube 30 may be opened.

Examples of the material for the hydrophilic resin film 40 covering the outer periphery of the resin tube 30 include resins having hydrophilic groups such as -OH, -CONH₂, -COOH, -NH₂, -COO⁻, and -SO₃⁻. Preferably, these resins have a functional group capable of linking to the surface of the resin tube 30. For example, polyvinylpyrrolidone, polyethylene glycol, and the like are advantageously adopted.

In order to form a linking structure between the resin tube 30 and the hydrophilic resin film 40, for example, the resin tube 30 uses a resin having a remnant isocyanate group or a resin reactive to an isocyanate group. If the resin tube 30 uses a resin reactive to an isocyanate group, a preferred practice comprises the steps of reacting with the resin a compound having an isocyanate group and linking via the isocyanate group a hydrophilic resin such as polyvinylpyrrolidone and polyethylene glycol. Details of the above-described method for forming the hydrophilic resin film 40 are disclosed in, for example, Japanese Patent Applications Laid-Open Nos. 5-184,666, 7-80,078, and 7-124,263.

The outer diameter C of the resin tube 30 and hydrophilic resin film 40 provided as described above is preferably smaller than the product outer diameter D in the main section 11b of the core 11. In this way, the diameter of the tip section of the guide wire 10 is made smaller, and as a result, the insertion even into fine blood vessels becomes possible. At the same time, the rigidity of the main section 11b of the core 11 can be maintained, and, as a result, the push ability at the time of insertion can be enhanced. The term "product outer diameter D" as used herein means the outermost diameter including the main section 11b of the core 11 and the hydrophobic resin film 12 and others provided thereon.

In the present invention, the metal coil 20 impermeable to X-rays can be replaced with a metal ring or tip having a similar function. Alternatively, the metal coil 20 can be omitted by blending a material impermeable to X-rays into the resin tube 30.

From the standpoint of slipperiness, it is basically preferable that the resin film which covers the guide wire is a hydrophilic resin film. However, it is difficult to cover the metal directly with a hydrophilic resin. The covering provided by the resin tube according to the present invention is so satisfactorily thin and excellent in formability that even the whole guide wire may be covered with the resin. In the embodiment, however, a hydrophobic resin film, which well meets the requirements of slipperiness and prevention of the adhesion of thrombi and which can form a very thin film, is directly applied to the main section of the core without further decreasing the outer diameter of the main section of the core. Owing to this construction, it is possible for the tip section, which needs to have a high-level lubricating property, to have a very good slipperiness due to the hydrophilic resin film, and, as a result, the push ability of the guide wire is enhanced.

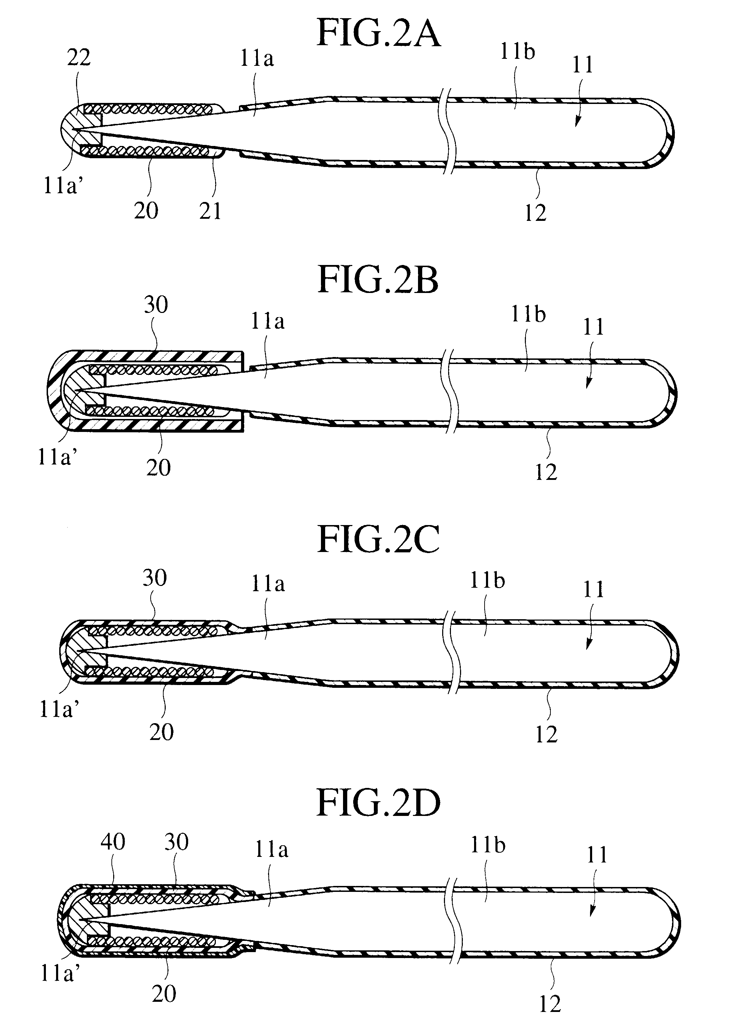

Figs. 2A to 2D illustrate an embodiment of the method for producing the clinical guide wire of the present invention. In these figures, the parts substantially the same as those of Fig. 1 are given the same symbols and the explanations thereof are omitted.

As shown in Fig. 2A, the diameter of the tip section 11a of a core 11 is dwindled, for example, taper-wise or stepwise by means of, for example, machining or etching. A metal coil 20, which is impermeable to X-rays, is fixed to the tip section 11a by means of soldering materials 21 and 22. The soldering material 22 is also fixed to the endmost tip 11a' of the core 11 to thereby form a round head. An adhesive may be used in place of the soldering materials 21 and 22. The outer periphery of the main section 11b of the core 11 is covered with a hydrophobic resin film 12 which is made from a silicone resin, a fluorocarbon resin, or the like.

Next, as shown in Fig. 2B, the outer periphery of the tip section 11a of the core 11 and the metal coil 20 is covered with a resin tube 30 which is swollen with a solvent. In this case, the inner diameter, which is originally smaller than that of the outer diameter of the metal coil 20, is swollen with a solvent to become larger than that of the outer diameter of the metal coil 20. Therefore, the swollen resin tube 30 can be easily provided on the outer periphery of the metal coil 20. If a polyurethane tube is used as the resin tube 30, preferred examples of the solvent to be used for the swelling include dichloromethane, methyl ethyl ketone (MEK), and toluene.

When the solvent of the resin tube 30 in the above-described state is evaporated, the resin tube 30 shrinks and adheres to the outer periphery of the metal coil 20. In this way, the outer periphery of the tip section 11a of the core 11 and the metal coil 20 is covered with the resin tube 30 as illustrated in Fig. 2C. In this case, the resin tube 30 has a uniform thickness and the degree of surface smoothness of the resin tube 30 is higher, relative to the resin surface obtained by other coating method such as extrusion or the like. In addition, since the core 11 can be covered with the resin tube 30 at room temperature without being heated, the characteristics of the core 11 made from an ultra-elastic material and the like are not impaired.

Then, as shown in Fig. 2D, a hydrophilic resin film 40 is formed on the outer periphery of the resin tube 30 by a method previously described. In this way, the clinical guide wire 10 can be produced.

Besides the above-described method for producing the clinical guide wire of the present invention, the following method can also be employed. That is, the outer periphery of the tip section 11a of the core 11 and the metal coil 20 is covered with a resin tube 30 whose inner diameter is made to a size capable of covering the foregoing outer periphery. Then, a portion of the resin tube 30, for example the inner periphery (e.g., 30a of Fig. 1) in the opening, is fixed to the core 11 by bonding or fusion. After that, a hydrophilic resin film 40 is formed on the outer periphery of the resin tube 30 by a method previously described.

In the above-described procedure, a urethane acrylate-based resin, a cyanoacrylate-based resin, an acrylate-based resin, or the like is used as the adhesive. The fusion can be performed using a solvent such as methyl ethyl ketone (MEK), tetrahydrofuran (THF), or the like. Alternatively, the adhesive may be a solution prepared by dissolving in the above-mentioned solvent a urethane resin or the like constituting a material for the resin tube.

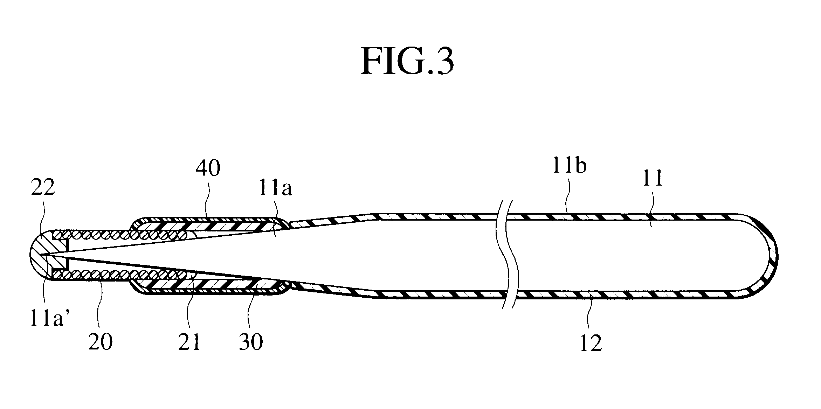

Fig. 3 illustrates another embodiment of the clinical guide wire of the present invention. In this figure, the parts substantially the same as those of Figs. 1 and 2 are given the same symbols and the explanations thereof are omitted.

According to this clinical guide wire, a resin tube 30 is provided to cover the portion ranging from a position in the vicinity of the base of the tip section lla of the core 11 to part of a metal coil 20. Therefore, this embodiment is distinguished in that the metal coil 20 and the head made from a soldering material 22 protrude from the tip of the resin tube 30. This construction, in which a portion on the tip side of the metal coil 20 and the head protrude from the tip of the resin tube 30, makes it possible to increase the flexibility of the tip section of the guide wire.

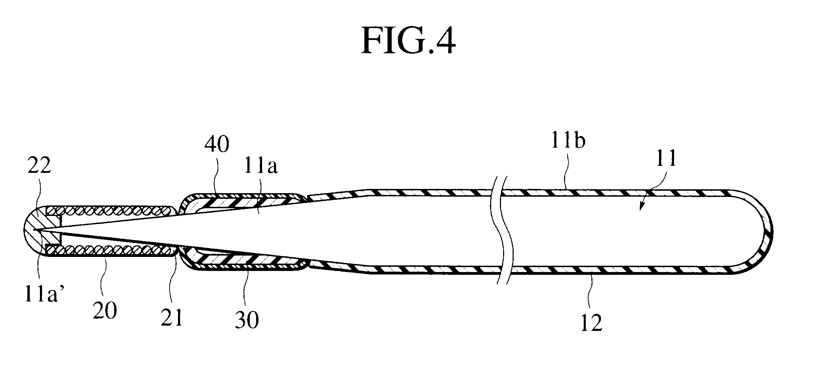

Fig. 4 illustrates another embodiment of the clinical guide wire of the present invention. In this figure, the parts substantially the same as those of Figs. 1 and 2 are given the same symbols and the explanations thereof are omitted.

This clinical guide wire is almost the same as the embodiment of Fig. 3. However, this embodiment is distinguished in that a resin tube 30 is provided to cover a portion ranging from a position in the vicinity of the base of the tip section 11a of the core 11 to a position short of the metal coil 20. This construction, in which whole of the metal coil 20 and the head protrude from the tip of the resin tube 30, makes it possible to further upgrade the flexibility of the tip of the guide wire.

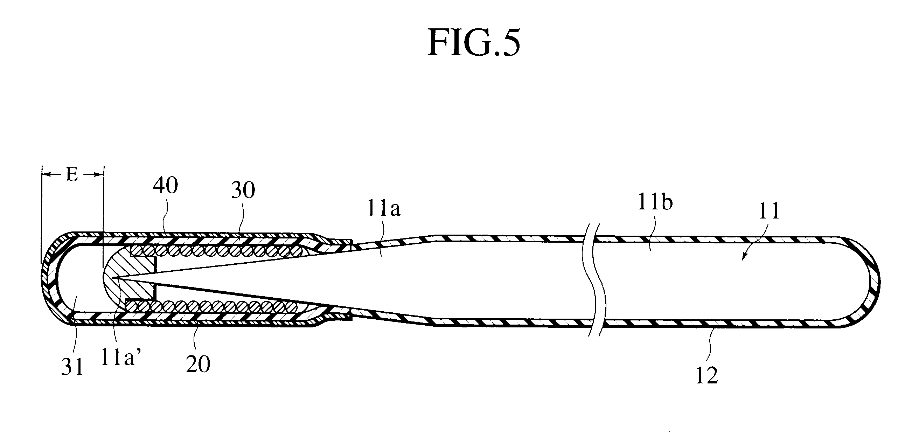

Fig. 5 illustrates another embodiment of the clinical guide wire of the present invention. In this figure, the parts substantially the same those of Figs. 1 and 2 are given the same symbols and the explanations thereof are omitted.

In this clinical guide wire, a resin tube 30 completely encloses the metal coil 20 attached to the tip section 11a of the core 11, wherein the tip of the resin tube 30 protrudes ahead of the endmost tip 11a' of the core 11 and the head formed therein. That is, the endmost tip of the guide wire is composed solely of the resin tube 30, wherein the interior 31 of the endmost tip of the resin tube 30 is preferably void. This construction enables the flexibility of the endmost tip of the guide wire to be further improved and provides better slipperiness as a result of complete enclosure of the metal coil 20 in the resin tube 30. The length of the tip, i.e., the length of E in Fig. 5, of the resin tube 30 protruded from the endmost tip 11a' of the core 11 and the head is preferably 2 to 20 mm.

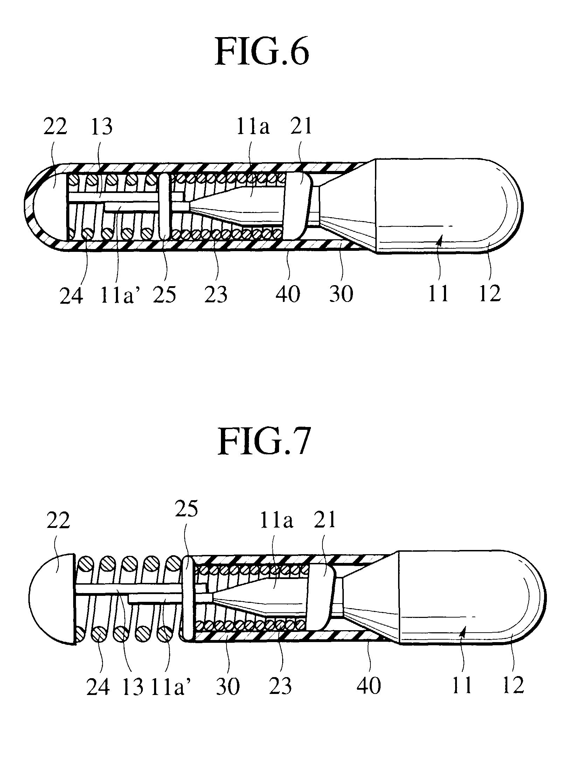

Fig. 6 illustrates another embodiment of the clinical guide wire of the present invention. In this figure, the parts substantially the same as those of Figs. 1 and 2 are given the same symbols and the explanations thereof are omitted.

In this clinical guide wire, the tip section lla of the core 11 has a diameter dwindled in two steps towards the tip, wherein to the endmost tip 11a' is linked a safety wire 13 by brazing or other means so that it extends forward. The safety wire 13 is made from a fine wire, such as a round or flat stainless steel wire, having a diameter smaller than that of the tip section 11a of the core 11, so that the tip section of the guide wire is made more flexible. The tip of the safety wire 13 is fixed to the soldering material 22 forming a round head.

On the outer periphery of the tip section 11a of the core 11, a first metal coil 23 is provided in the vicinity of the base thereof, while a second metal coil 24 is provided on the tip side thereof. The first metal coil 23 is preferably made from platinum or stainless steel. The base end of the first metal coil 23 is fixed to the core 11 by means of a soldering material 21 and the distal end is fixed to the core 11 and to the second metal coil 24 by means of a soldering material 25. The second metal coil 24 is made from a material such as platinum, impermeable to X-rays. The base end of the second metal coil 24 is fixed to the distal end of the first metal coil 23 and to the core 11 by means of a soldering material 25, while the distal end is fixed to a soldering material 22 forming a round head. The second metal coil 24 acts as a marker impermeable to X-rays and is given flexibility by spacing the pitch interval.

A resin tube 30 is provided to cover all of the tip section lla of the core 11, the first metal coil 23, the second metal coil 24, and the soldering material 22 which forms the round head. The surface of the resin tube 30 is covered with a hydrophilic resin film 40, as described previously.

In this clinical guide wire, the safety wire 13 is connected to the tip section 11a of the core 11, and the second metal coil 25 which acts also as a marker impermeable to X-rays is provided as an open-pitch coil. This construction can make the tip section of the guide wire flexible.

Fig. 7 illustrates yet another embodiment of the clinical guide wire of the present invention. In this figure, the parts substantially the same as those in the embodiment illustrated in Fig. 6 are given the same symbols and the explanations thereof are omitted.

The construction of this clinical guide wire is basically the same as that of the embodiment shown in Fig. 6. However, this embodiment is distinguished in that a resin tube 30 covers the outer periphery of the tip section 11a of the core 11 and the first metal coil 23 but the resin tube 30 does not cover the outer periphery of the second metal coil 24.

According to this clinical guide wire, since the resin tube 30 is not provided on the outer periphery of the second metal coil 24 and since the second metal coil 25 is an open-pitch coil, the tip section of the guide wire can be made more flexible.

### EXAMPLES

### Example 1

According to the manufacturing process shown in Figs. 2A to 2D, a guide wire was prepared. As a core 11, use was made of a stainless steel wire having an outer diameter of the main section 11b of 0.35 mm and a total length of 1800 mm. The portion ranging from the endmost tip 11a' to a length of 500 mm of the core 11 was tapered to thereby form a tip section lla.

A metal coil 20, made from a platinum alloy and impermeable to X-rays, was attached to the outer periphery of the tip section 11a by means of soldering materials 21 and 22, while the endmost tip 11a' of the corell was fixed to the soldering material 22 to thereby form a round head.

The outer periphery of the coil 20 was covered with a resin tube 30 which was made from polyurethane and swollen with methyl ethyl ketone. The resin tube 30 was fixedly attached to the tip section 11a of the corell and the outer periphery of the coil 20 by evaporating the solvent from the swollen tube.

The portion covered with the resin tube 30 was immersed in a methyl ethyl ketone solution containing 5% by weight of 4,4-diphenylmethane isocyanate for 30 to 120 seconds. Then, the above-mentioned portion was left to stand for 10 to 60 minutes at 60° C. In this way, unreacted isocyanate groups were formed on the surface of the resin tube 30.

Further, the portion covered with the resin tube 30 was immersed in a dichloromethane solution containing 2 to 10% by weight of polyvinylpyrrolidone having a molecular weight of 1,200,000 ("K-90" manufactured by Wako Pure Chemical Industries, Ltd.) for 3 to 60 seconds. Then, the above-mentioned portion was left to stand for 3 to 12 hours at 60°C so that the polyvinylpyrrolidone was linked to the unreacted isocyanate groups and, at the same time, the molecules of the polyvinylpyrrolidone were mutually polymerized. In this way, a hydrophilic resin film 40 was prepared.

The guide wire 10 thus obtained was used for the introduction of a catheter. The operational results were excellent, because the guide wire 10 exhibited good slipperiness inside the catheter and a blood vessel without the adhesion of thrombi on the surface and because the push ability at the time of insertion was good.

### Example 2

As in Example 1, a metal coil 20, impermeable to X-rays, was fixed to the outer periphery of the tip section lla of the corell by means of soldering materials 21 and 22. The outer periphery of the coil 20 was covered with a resin tube 30 which was made from polyurethane (without being swollen with a solvent) and the inner diameter of which was a littler larger than the outer diameter of the coil 20.

Then, the inner periphery 30a (see Fig. 1) in the opening of the resin tube 30 was fixedly attached to the core 11 by means of an acrylate-based UV-curable adhesive ("Three Bond 3018" manufactured by Three Bond Co., Ltd.).

Further, a hydrophilic resin film 40, made from polyvinylpyrrolidone, was formed on the outer periphery of the resin tube 30 by the same method as in Example 1. At the time when the hydrophilic resin film 40 was formed, the solvent softened the resin tube 30. Therefore, the resin tube 30 was stretched, because only the inner periphery 30a of the opening was fixed. As a result, the resin tube 30, which was thin, could be formed on the core. Next, the resin tube section protruding from the endmost tip was cut off and the tip of the resin tube was closed by means of the above-described adhesive.

The guide wire 10 thus obtained was also used for the introduction of a catheter. The operational results were excellent, because the guide wire 10 exhibited good slipperiness inside the catheter and a blood vessel without the adhesion of thrombi on the surface and because the push ability at the time of insertion was good.

The entire contents of Japanese Patent Applications P10-243228 (filed August 28, 1998) and P11-110948 (filed April 19, 1999) are incorporated herein by reference.

Although the invention has been described above by reference to certain embodiments of the invention, the invention is not limited to the embodiments described above. Modifications and variations of the embodiments descried above will occur to those skilled in the art, in light of the above teachings. The scope of the invention is defined with reference to the following claims.

## Claims

1. A clinical guide wire, comprising:
a core made from an elastic material such that the tip section has a diameter smaller than that of the main section;
a resin tube which covers at least part of the tip section of the core; and
a hydrophilic resin film which covers the outer periphery of the resin tube,
wherein the outer diameter of the portion covered with the resin tube and the hydrophilic resin film is smaller than the product outer diameter in the main section of the core.

2. A clinical guide wire according to claim 1,
wherein a metal coil impermeable to X-rays is attached to the outer periphery of the endmost tip of the core; and
wherein the outer periphery of the metal coil is covered completely with the resin tube.

3. A clinical guide wire according to claim 1,
wherein a metal coil impermeable to X-rays is attached to the outer periphery of the endmost tip of the core; and
wherein at least the tip of the metal coil protrudes from the resin tube.

4. A clinical guide wire according to claim 1,
wherein the resin tube covers the tip section of the core such that the resin tube further protrudes from the endmost tip of the core.

5. A clinical guide wire according to any one of claims 1 to 4,
wherein the main section of the core is covered with a hydrophobic resin film.

6. A clinical guide wire according to any one of claims 1 to 5,
wherein a material impermeable to X-rays is blended into the resin tube for the core.

7. A method for producing a clinical guide wire, comprising the steps of:
(a) making a core from an elastic material such that the diameter of the tip section is smaller than that of the main section;
(b) covering at least part of the tip section of the core with a resin tube; and
(c) covering the outer periphery of the resin tube with a hydrophilic resin film.

8. A method for producing a clinical guide wire according to claim 7,
wherein the resin tube which covers at least part of the tip section of the core is a resin tube swollen with a solvent; and
wherein the method for producing a clinical guide wire further comprises the following step after the step (b) and before the step (c):
evaporating the solvent to shrink-wrap the tip section of the core with the resin tube.

9. A method for producing a clinical guide wire according to claim 7, further comprising the step of, after the step (b) and before the step (c):
bonding or fusing the resin tube to the tip section of the core.

10. A method for producing a clinical guide wire according to any one of claims 7 to 9, further comprising the step of, after the step (a) and before the step (b):
attaching a metal coil impermeable to X-rays to the outer periphery of the endmost tip of the core,
wherein the outer periphery of the metal coil is then covered with the resin tube in the following step (b).

11. A clinical guide wire (10) comprising: an elongate member (11) having a first portion (11b) and an end portion (11a); a resinous layer (30) covering at least part of said end portion (lla) and a hydrophilic film (40) covering the outer surface of the resinous layer (30), the outer diameter (C) of the hydrophilic film (40) about the end portion (11a) being less than the diameter (D) of the first portion (11b).

12. A method of manufacturing a clinical guide wire (10) comprising the steps of: providing an elongate member (11) having a first portion (11b) of substantially uniform diameter and having an end portion (11a); covering at least part of the end portion (11a) with a resinous layer (30); and covering an outer surface of the resinous layer (30) with a hydrophilic film (40), the outer diameter (C) of the film about the end portion (lla) being less than the diameter (D) of the first portion (11b)

13. A method as claimed in claim 12, wherein the step of covering at least part of the end portion (11a) with a resinous layer (30) comprises the step of swelling the resinous layer (30) with solvent and the step of evaporating the solvent to reduce the layer (30) to form an interference fit on said at least part of the end portion (11a).
